# EUROPÄISCHE PATENTSCHRIFT

(11) **EP 3 260 153 B1**
(45) Veröffentlichungstag und Bekanntmachung des Hinweises auf die Patenterteilung: **09.10.2024**
(21) Anmeldenummer: 17001043.3
(22) Anmeldetag: 21.06.2017
(51) Int. Cl.: A61M 16/00

(54) **BEATMUNGSGERÄT UND VERFAHREN**
VENTILATION DEVICE AND METHOD
RESPIRATEUR ET PROCÉDÉ

(30) Priorität: 22.06.2016 DE 102016007551
(43) Veröffentlichungstag der Anmeldung: 27.12.2017
(73) Patentinhaber: Löwenstein Medical Technology S.A., 2557 Luxembourg (LU)
(72) Erfinder: Dömer, Benno, 76275 Ettlingen (DE); Alshut, Rüdiger, 76131 Karlsruhe (DE); Schwaibold, Matthias, 76228 Karlsruhe (DE)
(74) Vertreter: Marx, Thomas

(56) Entgegenhaltungen:
- EP-A2- 1 023 912
- US-A1- 2016 058 962
- IEEE COMPUTER SOCIETY: "IEEE Standard for Information technology- Telecommunications and information exchange between systems- Local and metropolitan area networks- Specific requirements Part 11: Wireless LAN Medium Access Control (MAC) and Physical Layer (PHY) specifications Amendment 8: Medium Access Control (MAC) Qualit", IEEE STD IEEE STD IEEE STD IEEE STD IEEE STD IEEE STD IEEE STD IEEE STD, 11 November 2005 (2005-11-11), pages 11 - 211, XP055411192, Retrieved from the Internet <URL:http://ieeexplore.ieee.org/ielx5/10328/32891/01541572.pdf> [retrieved on 20170928]

## Beschreibung

Die vorliegende Erfindung betrifft eine Datenverarbeitungseinrichtung eines Beatmungsgerätes und ein Verfahren zum Betreiben einer Datenverarbeitungseinrichtung eines Beatmungsgerätes. Es werden Therapiedaten registriert und in wenigstens einer Speichereinrichtung der Datenverarbeitungseinrichtung hinterlegt. Wenigstens ein Teil der Therapiedaten wird mit wenigstens einer Übertragungseinrichtung an ein Netzwerk übertragen.

Heimbeatmungsgeräte bzw. Schlaftherapiegeräte verfügen in der Regel über eine Speichereinrichtung, um Therapiedaten hinterlegen und abrufen zu können. So werden in der Speichereinrichtung in der Regel Daten hinterlegt, die eine individuelle Einstellung des Beatmungsgerätes an die jeweiligen Bedürfnisse des Benutzers ermöglichen. Das betrifft beispielsweise die zur Therapie einer bestimmten Atemstörung notwendigen Beatmungsparameter.

Des Weiteren werden häufig auch Daten gespeichert, welche den Therapieverlauf charakterisieren. Solche Daten sind besonders hilfreich, um einen Erfolg der Therapie beurteilen zu können oder um eine bestimmte Diagnose zu unterstützen. Zudem liefern die Daten auch Auskunft darüber, ob der Benutzer das Beatmungsgerät korrekt und regelmäßig anwendet.

Um eine zuverlässige Diagnose und eine wirksame Therapie gewährleisten zu können, ist daher eine regelmäßige bzw. möglichst häufige Beurteilung der Therapiedaten besonders wichtig. In der Regel erfolgt die Begutachtung der Therapiedaten im Rahmen eines Besuchs des Betreuers. Das hat allerdings den Nachteil, dass zwischen den Besuchen keine Begutachtung der Therapiedaten stattfindet. Zudem sind Besuche mit einem erheblichen Kostenaufwand verbunden.

Im Stand der Technik sind daher Beatmungsgeräte bekannt geworden, welche sich über ein GSM-Modem in ein Mobilfunknetz einwählen und darüber die Therapiedaten an ein Netzwerk übertragen. Die Begutachtung der Therapiedaten kann dadurch an einer zentralen Stelle unabhängig vom Ort des Benutzers und zu einer beliebigen Zeit erfolgen. US 2016/0058962 A1 offenbart ein Beatmungsgerät mit einer Datenverarbeitungseinrichtung, die Therapiedaten registriert und in einer Speichereinrichtung hinterlegt und wenigstens einen Teil der hinterlegten Therapiedaten an ein Netzwerk überträgt.

Die IEEE Computer Society offenbart in der Publikation "IEEE Standard for Information technology- Telecommunications and information exchange between systems- Local and metropolitan area networks- Specific requirements Part 11: Wireless LAN Medium Access Control (MAC) and Physical Layer (PHY) specifications Amendment 8: Medium Access Control (MAC) Quality of Service Enhancements" (pages 11 - 211, URL: http://ieeexplore.ieee.org/ielx5/10328/32891/01541572.pdf) vom 11.11.2005 Standards im Bereich der lokalen drahtlosen Netze (WLAN) sowie Protokolle und Maßnahmen, die die Dienstgüte des Kommunikationsdienstes aus der Sicht der Anwender beeinflussen.

Allerdings bietet diese Übertragung der Daten erhebliche Nachteile. So fallen für die Übertragung mittels eines GSM-Modems in der Regel Kosten eines Netzanbieters an. In der Praxis hat sich gezeigt, dass diese Kosten sowohl vom Benutzer als auch von einem Kostenträger und beispielsweise einem Versicherer häufig sehr unerwünscht sind.

Nachteilig ist zudem, dass die Übertragung mit einem GSM-Modem oft sehr energieaufwendig ist. Das wirkt sich besonders nachteilig bei Beatmungsgeräten mit einem netzunabhängigen Akkubetrieb aus. Ein weiterer Nachteil ist, dass die Benutzer der von einem GSM-Modem ausgehenden elektromagnetischen Strahlungsleistung oft sehr kritisch gegenüberstehen.

Es ist daher die Aufgabe der vorliegenden Erfindung, eine Datenaufbereitungseinrichtung eines Beatmungsgerätes und ein solches Beatmungsgerät zur Verfügung zu stellen, welche eine verbesserte und insbesondere eine kostenoptimierte und/oder energieoptimierte Übertragung von Therapiedaten an ein Netzwerk ermöglichen.

Diese Aufgabe wird mit Anspruch 1 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die erfindungsgemäße Datenverarbeitungseinrichtung eines Beatmungsgerätes und insbesondere eines Heimbeatmungsgerätes und/oder Schlaftherapiegerätes. Ein Beatmungsgerät kann mit einer erfindungsgemäßen Datenverarbeitungseinrichtung betrieben werden. Anhand der Datenverarbeitungseinrichtung werden Therapiedaten registriert und in wenigstens einer Speichereinrichtung der Datenverarbeitungseinrichtung hinterlegt. Wenigstens ein Teil der hinterlegten Therapiedaten wird mit wenigstens einer Übertragungseinrichtung an wenigstens ein Netzwerk übertragen. Dabei wird mit der Übertragungseinrichtung wenigstens ein verfügbares Netz ermittelt. Mit der Übertragungseinrichtung wird wenigstens eine Kenngröße für eine Netzqualität des Funknetzes bestimmt. Mit der Datenverarbeitungseinrichtung wird wenigstens eine Aufbereitung der Therapiedaten vor ihrer Übertragung in das Funknetz in Abhängigkeit der Kenngröße dynamisch vorgenommen. Dadurch ist vorzugsweise eine zuverlässige und insbesondere verlustarme Übertragung der Therapiedaten auch bei einer schlechteren Netzqualität und insbesondere bei einer Verbindung zu einem Niedrigenergie-Hochreichweitennetz möglich.

Die erfindungsgemäße Datenverarbeitungseinrichtung hat viele Vorteile. Ein erheblicher Vorteil kann sein, dass eine dynamische Aufbereitung der Therapiedaten in Abhängigkeit der Netzqualität vorgenommen wird. Durch eine solche Aufbereitung der Therapiedaten ist ein zuverlässiges und sicheres Übermitteln auch an Funknetze mit einer eingeschränkten Verfügbarkeit bzw. Übertragungsqualität möglich. Dadurch können zur Übertragung auch Funknetze herangezogen werden, welche eine kostenfreie oder besonders kostengünstige und energieoptimierte
Übertragung der Therapiedaten ermöglichen. Beispielsweise können die Therapiedaten aufgrund der erfindungsgemäßen Aufbereitung auch in Niedrigenergie-Hochreichweitennetze (Low Power Wide Area Network, ZPWAN) oder andere kosten- und energieoptimierte Funknetze übertragen werden.

Da solche Funknetze erheblich andere Anforderungen als beispielsweise die üblichen Mobilfunknetze bzw. GSM-Netze erfordern, ist die Aufbereitung der Therapiedaten dabei besonders entscheidend. Mit der hier vorgestellten Datenverarbeitungseinrichtung kann beispielsweise die Anforderung erfüllt werden, dass die Datenmengen sehr kompakt sein müssen. Zudem kann durch die erfindungsgemäße Aufbereitung gewährleistet werden, dass die versendeten Therapiedaten auch vollständig ihr jeweiliges Ziel im Funknetz erreichen.

Da die üblichen Mobilfunknetze hinsichtlich der Übertragungsgeschwindigkeit und des Übertragungsvolumens optimiert sind, ist eine Anpassung der Therapiedaten in Abhängigkeit der Netzqualität bei solchen Funknetzen in der Regel nicht notwendig. Allerdings bieten derartige Mobilfunknetze den Nachteil, dass sie sehr energiehungrig sind und einen unerwünschten Kostenaufwand mit sich bringen.

Vorzugsweise kann die Kenngröße eine Verfügbarkeit des Funknetzes c.efiniert. Bevorzugt definiert die Kenngröße eine Übertragungszuverlässigkeit des Netzes. Die Kenngröße kann dazu beispielsweise eine Signalstärke beschreiben. Möglich sind auch andere Kenngrößen, welche für eine Definition der Netzqualität geeignet sind. Anhand solcher Kenngrößen können die Therapiedaten besonders gut aufbereitet werden, um eine möglichst vollständige und zuverlässige Übertragung zu gewährleisten.

Möglich ist auch, dass die Kenngröße wenigstens einen Kostenfaktor der Nutzung des Funknetzes beschreibt. Eine solche Kenngröße kann besonders gut zur kostenoptimierten Datenübertragung herangezogen werden. Es ist auch möglich, dass das die Kenngröße wenigstens ein Maß für den Energieaufwand für eine Übertragung in das Netz beschreibt. In Abhängigkeit einer solchen Kenngröße kann eine besonders gezielte Energieoptimierung der Übertragung vorgenommen werden. Das ist beispielsweise dann von Vorteil, wenn die Übertragung unabhängig von einem elektrischen Stromnetz erfolgen soll.

Besonders bevorzugt kann mit der Übertragungseinrichtung wenigstens ein Signal-Rausch-Abstand (SNR) und/oder wenigstens eine Signalstärke im Uplink und/oder Downlink und/oder wenigstens ein RSSI (Received Signal Strength Indicator) als Kenngröße für die Netzqualität bestimmt werden. Möglich ist auch, dass die Kenngröße wenigstens ein Ergebnis einer Listenbefore-Talk Abfrage und/oder eine Belegung/Traffic in dem jeweiligen Funknetz beschreibt.

Bevorzugt kann wenigstens eine vom Netzbetreiber zur Verfügung gestellte Kenngröße für die Netzqualität ermittelt und zur dynamischen Aufbereitung der Therapiedaten herangezogen werden.

Vorzugsweise könnte mit der Übertragungseinrichtung wenigstens eine der nachfolgend aufgeführten Kenngrößen für die Netzqualität bestimmt werden: Link Budgets, Anzahl verfügbarer und/oder verbundener Base Stations des Funknetzes, Abstand zur nächsten Base Station, Rauschniveaus und/oder Fremdsignalquellenpegel und/oder Kanalbelegungen in den Übertragungsbereichen, Übertragungslatenzen, durch die Übertragungseinrichtung und/oder Middieware/Anwendungsserver und/oder weitere Systemkomponenten gemessene und/oder errechnete Kenngrößen, statistische Kenngrößen aus bisherigen Übertragungen, Anteil und/oder Anzahl erfolgreicher oder vergeblicher Verbindungs- und Rekonnektionsversuche, Anzahl detektierter Kollisionen von Datenpaketen.

Die Kenngröße kann auch wenigstens eine vom Funknetz bzw. dem Niedrigenergie-Hochreichweitennetz gewährte und/oder verlangte und/oder ausgehandelte Übertragungsratenstärke und/oder Übertragungssignalstärke beschreiben. Die Kenngröße kann auch antizipierte Störungslevel und/oder Auslastungslevel definieren, wie z. B. tageszeitliche Schwankungen.

Bevorzugt können die Therapiedaten zur Aufbereitung priorisiert werden. Durch eine Priorisierung kann das zu übertragende Datenvolumen erheblich reduziert und insbesondere an Niedrigenergie-Hochreichweitennetze angepasst werden. Insbesondere werden vorrangig und/oder ausschließlich die Therapiedaten übertragen, welche wenigstens einen Schwellenwert für eine Priorität überschreiten. Im Rahmen der vorliegenden Erfindung kann je nach Definition eines Schwellenwertes auch vorgesehen sein, dass vorrangig und/oder ausschließlich Therapiedaten übertragen werden, welche wenigstens einen Schwellenwert für eine Priorität unterschreiten.

Es ist möglich, dass die Priorität unter Berücksichtigung einer therapeutischen und/oder diagnostischen Funktion der entsprechenden Therapiedaten zugeordnet werden kann. Beispielsweise kann einem zur gewünschten Beatmung benötigten Geräteparameter eine höhere Priorität für die Übertragung zugeordnet werden. Beispielsweise wird eine besonders hohe Priorität zugeordnet, wenn die Therapiedaten die Zuverlässigkeit der Behandlung und/oder der Diagnose betreffen. Möglich ist auch, dass die Priorität in Abhängigkeit der Bedeutung der Therapiedaten für eine Betriebszuverlässigkeit des Beatmungsgerätes zugeordnet wird. Die Priorität kann auch in Abhängigkeit der Bedeutung der Therapiedaten für eine Abrechnung und/oder einen Nutzungsnachweis zugeordnet werden. Anhand eines Abgleichs der Priorität mit dem Schwellenwert werden solche Daten dann vorzugsweise vorrangig und/oder ausschließlich übertragen.

Besonders bevorzugt kann die Priorität in Abhängigkeit einer Veränderung der Therapiedaten zugeordnet werden. Beispielsweise kann dazu der Zeitpunkt einer letztmaligen Änderung herangezogen werden. Dadurch müssen unveränderte Daten nicht übertragen werden und das Datenvolumen kann somit entsprechend gering gehalten werden. Zudem kann so ein besonders sicherer Betrieb gewährleistet werden, da Veränderungen der Therapiedaten sofort erkannt und bei der Therapie berücksichtigt werden können. So kann anhand der Daten schnell festgestellt werden, ob ein Benutzer Veränderungen an der Gerätekonfiguration vorgenommen hat oder ob er verändert auf die Beatmung reagiert.

Einer Therapiestundenanzahl und/oder einem Therapieverlauf und/oder einer Therapiestatistik kann besonders bevorzugt eine Priorität oberhalb des Schwellenwertes zugeordnet werden. Insbesondere wird einem Therapieverlauf und/oder einer Therapiestatistik des aktuellen und/oder des vorherigen Tages und/oder des zuletzt vom Netzwerk empfangenen Tages eine Priorität oberhalb des Schwellenwertes zugeordnet. Ergänzend ist auch möglich, dass solche Daten mit einer erhöhten Redundanz übertragen werden. Möglich ist auch, dass Störungsmeldungen und/oder Fehlerberichte eine Priorität oberhalb des Schwellenwertes zugeordnet bekommen. Solche Daten sind zur Beurteilung der Therapie bzw. zur Erstellung einer Diagnose besonders aussagekräftig. Durch die erhöhte Priorität kann daher eine Übermittlung auch bei schwächerem Funknetz gewährleistet werden.

Der Schwellenwert für die Priorität kann vorzugsweise in Abhängigkeit der Kenngröße dynamisch festgelegt werden. Beispielsweise kann der Schwellenwert für die Priorität erhöht werden, wenn die Kenngröße eine schlechte Netzqualität definiert. Dann werden vorzugsweise die Therapiedaten vorrangig oder ausschließlich übertragen, welche eine sehr hohe Priorität aufweisen. So ist gewährleistet, dass die zur Verfügung stehende Netzqualität ausschließlich oder im Wesentlichen zur Übertragung der wichtigen Daten verwendet wird. Vorzugsweise wird der Schwellenwert herabgesetzt, wenn die Kenngröße eine stabile und hohe Datenübertragung für das Netz definiert. In einem solchen Fall können dann auch weniger vorrangige Daten übertragen werden, wie zum Beispiel langfristige Statistikdaten.

Die Therapiedaten können vorzugsweise mit einer Redundanz übertragen werden. Die Redundanz wird vorzugsweise in Abhängigkeit der Kenngröße dynamisch eingestellt. Ergänzend wird in Abhängigkeit der Kenngröße die Anzahl der Wiederholungen der Übertragung wenigstens eines Teils der Therapiedaten eingestellt. Beispielsweise kann eine Mehrzahl von Wiederholungen vorgesehen sein, welche über einen Tag oder über eine Woche oder über einen Monat oder einen anderen Zeitraum verteilt sind. Die Therapiedaten werden vorzugsweise zu verschiedenen Tageszeiten und/oder während einer Therapie redundant übertragen. Es kann auch eine redundante Übertragung vorgesehen sein, bei welcher die Redundanz unabhängig von der Kenngröße eingestellt wird.

Besonders bevorzugt werden die Wiederholungen wenigstens so lange fortgesetzt, bis wenigstens eine Empfangsbestätigung als Downlink vom Netz erhalten wird. Möglich ist auch, dass die Wiederholungen unabhängig von einer Empfangsbestätigung wiederholt werden können. Insbesondere kann dabei eine Maximalzahl an Wiederholungen vorgesehen sein. Vorzugsweise wird die Maximalzahl in Abhängigkeit der Kenngröße dynamisch eingestellt.

Es kann bevorzugt möglich sein, dass ein Teil der Therapiedaten mit einer höheren Redundanz als ein anderer Teil der Therapiedaten übertragen wird. Vorzugsweise werden Therapiedaten, welche einen Schwellenwert für eine Priorität überschreiten, mit einer höheren Redundanz übertragen.

Insbesondere werden diese Therapiedaten mit einer höheren Redundanz als Therapiedaten, welche den Schwellenwert für eine Priorität unterschreiten, übertragen. Eine solche Kombination aus Priorität und Redundanz ermöglicht eine besonders zuverlässige Übertragung wichtiger Therapiedaten. Insbesondere erfolgt die Anpassung der Redundanz sowohl in Abhängigkeit der Kenngröße für die Netzqualität als auch unter Berücksichtigung der Priorität.

In einer vorteilhaften Weiterbildung kann in Abhängigkeit der Kenngröße festgelegt werden, dass die Therapiedaten über wenigstens eine andere Netzverbindung als das Funknetz übertragen werden. Die Übertragungseinrichtung verbindet sich dann insbesondere über wenigstens ein GSM-basiertes Modem mit wenigstens einem Mobilfunknetz. Die andere Netzverbindung ist insbesondere für eine Übertragungsgeschwindigkeit und für ein Datenvolumen optimiert. Die andere Netzverbindung ist vorzugsweise ein Funknetz ohne eine Energieoptimierung, wie sie für das Niedrigenergie-Hochreichweitennetz vorgesehen ist.

So kann bei einer ungünstigen Netzqualität des Funknetzes eine sichere und zuverlässige Übertragung von wichtigen Daten über die andere Netzverbindung gewährleistet werden. Das ist beispielsweise dann der Fall, wenn für einen Betrieb des Beatmungsgerätes ein schneller Downlink benötigt wird, beispielsweise bei einer Ferneinstellung oder einer Neukonfiguration.

Ergänzend könnte auch möglich sein, dass die andere Netzverbindung durch wenigstens ein lokales Netz und beispielsweise ein WLAN (Wireless Local Area Network) oder LAN (Local Area Network) zur Verfügung gestellt wird. Beispielsweise werden die Daten in Abhängigkeit der Kenngröße über das lokale Netz auf ein Netzwerkgerät des Patienten und/oder Betreuers übertragen. Von dort werden die Therapiedaten insbesondere über ein Hochreichweitennetz bzw.

Weitverkehrsnetz beispielsweise weiter zu einer Auswerteeinrichtung zur therapeutischen und/oder diagnostischen Analyse übertragen. Beispielsweise erfolgt dies per Internet, VPN und/oder eine Mobilfunkverbindung. Möglich ist aber auch, dass die Therapiedaten direkt über ein Hochreichweitennetz übertragen werden, welches nicht energieoptimiert ist.

Es kann auch vorgesehen sein, dass in Abhängigkeit der Kenngröße die Therapiedaten auf wenigstens einem Speichermedium und insbesondere auf einem transportablen Speichermedium gespeichert werden. Das transportable Speichermedium ist insbesondere austauschbar und/oder entnehmbar im Beatmungsgerät angeordnet. Das Speichermedium umfasst z. B. einen Flash-Speicher oder ist als ein solcher ausgebildet. Das ist besonders bei sehr großen Datenmengen von Vorteil. Das Speichermedium kann dann beispielsweise einem Betreuer und/oder einer Auswerteeinrichtung auf dem Postweg zugestellt werden.

Eine andere alternative Netzverbindung, welche insbesondere nicht energieoptimiert ist, kann eine zuverlässige Möglichkeit bieten, auch größere Datenmengen schnell übertragen zu können. Da eine solche Netzverbindung in der Regel mit höheren Kosten und/oder einem höheren Energieaufwand verbunden ist, ist diese vorzugsweise als Ausweichmöglichkeit bei einem energieoptimierten Funknetz mit ungünstiger Kenngröße vorgesehen. Besonders bevorzugt ist daher eine kcstenoptimierte Auswahl vorgesehen, bei der die andere Netzverbindung und beispielsweise eine GSM-basierte Netzverbindung nur in Ausnahmefällen vorgesehen sind.

Möglich ist auch, dass die andere Netzverbindung bevorzugt genutzt werden kann, wenn ein Downlink und/oder Uplink mit erhöhtem Datenvolumen vorgesehen ist. Das ist beispielsweise dann der Fall, wenn ein Softwareupdate und/oder eine neue Gerätekonfiguration auf das Beatmungsgerät übertragen werden soll.

In einer Ausgestaltung der erfindungsgemäßen Datenverarbeitungseinrichtung kann in Abhängigkeit der Kenngröße wenigstens eine Ferneinstellbarkeit des Beatmungsgerätes eingeschränkt und/oder erweitert werden. Vorzugsweise wird eine Ferneinstellbarkeit eingeschränkt, wenn die Kenngröße eine niedrige Netzqualität definiert. Vorzugsweise wird eine Ferneinstellbarkeit erweitert, wenn die Kenngröße eine höhere Netzqualität definiert. Es ist möglich, dass die Ferneinstellbarkeit in Abhängigkeit der Kenngröße dauerhaft und/oder vorübergehend geändert wird. Es ist möglich, dass in Abhängigkeit der Kenngröße wenigstens ein Downlink übertragen wird, durch den die Ausführung der Änderung der Ferneinstellbarkeit veranlasst wird.

Unter einer Ferneinstellbarkeit wird insbesondere ein Rahmen verstanden, in dem das Beatmungsgerät über wenigstens eine Ferneinstellung bzw. Telesetting einstellbar ist. Eine Ferneinstellung ist insbesondere dadurch gekennzeichnet, dass Therapiedaten von einem anderen Ort als dem des Beatmungsgerätes an das Beatmungsgerät übermittelt werden, welche eine Einstellung wenigstens eines Geräteparameters und/oder eine Veränderung der Gerätekonfiguration und/oder einer Gerätesoftware bewirken. Eine Einschränkung der Ferneinstellbarkeit führt somit vorzugsweise dazu, dass nur noch bedingt Ferneinstellungen vorgenommen werden können.

Für eine Ferneinstellung ist eine hohe Netzqualität in der Regel sehr wichtig, da eine unvollständige Übertragung der Ferneinstellungsdaten ungültige bzw. fehlerhafte Konfigurationen hervorrufen kann. Eine Einschränkung der Ferneinstellbarkeit in Abhängigkeit der Kenngröße kann daher einen zuverlässigen Betrieb gewährleisten.

Wird die Ferneinstellbarkeit eingeschränkt, kann das Beatmungsgerät insbesondere auf alternative Gerätekonfigurationen in der Speichereinrichtung zurückgreifen.

So kann wenigstens teilweise unabhängig von der Ferneinstellung die Therapie zuverlässig durchgeführt werden. Es kann abgewartet werden, bis die Kenngröße eine verbesserte Netzqualität definiert, damit die Ferneinstellbarkeit wieder erweitert wird.

Es kann vorgesehen sein, dass in Abhängigkeit der Kenngröße wenigstens eine andere Netzverbindung als das Funknetz für die Ferneinstellung ausgewählt wird. Das ist beispielsweise dann der Fall, wenn eine Ferneinstellung gewünscht ist und die Kenngröße auf eine nicht ausreichende Netzqualität hinweist. Dann kann beispielsweise eine benötigte Gerätekonfiguration und/oder wenigstens ein Geräteparameter über die andere Netzverbindung gesendet werden.

Es ist möglich, dass die Kenngröße für die Netzqualität des Netzes durch wenigstens eine Benutzereingabe und/oder durch wenigstens eine Ferneinstellung festgelegt werden kann. Beispielsweise kann der Benutzer wenigstens eine Ortsangabe des Beatmungsgerätes vornehmen, in deren Abhängigkeit die Kenngröße festgelegt wird. Möglich ist auch, dass der Einsatzort über wenigstens eine Ferneinstellung in der Datenverarbeitungseinrichtung hinterlegt wird. Der Einsatzort des Beatmungsgerätes kann auch durch das Gerät selbst ermittelt werden, beispielsweise über ein Abhören des Netzes (Listen Before Talk) und/oder über Empfang eines GPS-Signals. Durch solche Angaben kann die an dem Einsatzort verfügbare Netzqualität besonders zuverlässig ermittelt werden und als Kenngröße herangezogen werden.

In allen Ausgestaltungen kann es besonders bevorzugt sein, dass die Kenngröße als wenigstens ein Downlink an das Beatmungsgerät übertragen wird. Insbesondere wird die Kenngröße als ein Downlink von wenigstens einer Basisstation des Netzes an das Beatmungsgerät übertragen. Das ist besonders von Vorteil, da dem Netz bzw. der Basisstation die Kenngröße häufig bekannt ist. Dadurch kann auf eine technisch aufwendige Ermittlung der Kenngröße durch das Beatmungsgerät verzichtet werden. Es ist möglich, dass die als Downlink ermittelte Kenngröße durch wenigstens einen Algorithmus in eine für das Beatmungsgerät verwendbare Kenngröße umgerechnet wird.

Bevorzugt können die Therapiedaten zur Aufbereitung auf Änderungen überprüft werden. Es ist möglich, dass die Therapiedaten dazu mit wenigstens einem älteren bzw. vorhergehenden Datensatz verglichen werden, welcher in der Speichereinrichtung hinterlegt ist. Möglich ist auch, dass die Therapiedaten auf Änderungen gegenüber Therapiedaten überprüft werden, welche in einer vorhergehenden Übertragung an das Netz übermittelt wurden. Beispielsweise kann ein Abgleich mit einer älteren Dateiversion vorgesehen sein. Besonders bevorzugt werden Gerätekonfigurationen und/oder Geräteparameter und/oder ein Therapieverlauf auf Änderungen überprüft. Das ist besonders vorteilhaft, da Gerätekonfigurationen bzw. Geräteparameter in der Regel nur dann für eine Auswertung relevant sind, wenn diese auch geändert wurden.

Vorzugsweise können Therapiedaten mit einem Datenvolumen oberhalb eines Schwellenwertes über wenigstens eine andere Netzverbindung als das Funknetz übertragen werden. Dabei ist insbesondere das Volumen eines zusammenhängenden Datenpakets und vorzugsweise eines Uplinks gemeint. Möglich ist auch, dass Therapiedaten mit einem Datenvolumen oberhalb eines Schwellenwertes auf wenigstens einem transportablen Speichermedium gespeichert werden. Es kann auch vorgesehen sein, dass Therapiedaten mit einer Priorität oberhalb eines Schwellenwertes über wenigstens eine andere Netzverbindung als das Funknetz übertragen werden.

Besonders bevorzugt könnte vorgesehen sein, dass zu empfangende Therapiedaten und insbesondere Downlinks mit einem Datenvolumen oberhalb eines Schwellenwertes über wenigstens eine andere Netzverbindung als das Funknetz übertragen werden. Das ist besonders vorteilhaft für Updates von Gerätesoftware und/oder Gerätekonfigurationen, da diese in der Regel ein hohes Datenvolumen aufweisen. Für derartige Updates kann auch vorgesehen sein, dass eine aktuellere Version der Gerätesoftware und/oder Gerätekonfiguration auf einem transportablen Speichermedium gespeichert ist.

In einer Ausgestaltung könnte es möglich sein, dass von dem Funknetz eine Empfangsbestätigung über die empfangenen Therapiedaten nur nach Ablauf eines vorbestimmten Zeitraums versendet wird. Möglich ist auch, dass von dem Funknetz eine Empfangsbestätigung über die empfangenen Therapiedaten nur in Verbindung mit wenigstens einer weiteren Empfangsbestätigung versendet wird und/oder wenigstens eines weiteren Downlinks von Therapiedaten versendet wird. Dadurch kann die Anzahl der zu versendenden Empfangsbestätigungen erheblich eingeschränkt werden, sodass das Datenvolumen der Downlinks eingeschränkt werden kann.

Besonders bevorzugt können zu versendende Empfangsbestätigungen über einen bestimmten Zeitraum gesammelt und nach einem bestimmten Zeitraum und/oder bei Erreichen einer bestimmten Anzahl gebündelt versendet werden. Eine vorteilhafte Optimierung des Übertragungsvolumens bietet auch die Kopplung einer zu versendenden Empfangsbestätigung mit wenigstens einem anderen Downlink. Beispielsweise wird eine Empfangsbestätigung mit einer zu versendenden Gerätekonfiguration und/oder einem Geräteparameter gekoppelt.

Zur Aufbereitung der Therapiedaten kann von der Datenverarbeitungseinrichtung wenigstens ein Schritt aus einer Gruppe von Schritten vorgenommen werden, umfassend: Datenkomprimierung, Einschränkung wenigstens eines Wertebereichs, Differenzierung und/oder Integration von Absolutwerten. Möglich ist auch, dass wenigstens eine Bitmaske angewendet wird. Zudem werden zur Aufbereitung bevorzugt auch eine Priorisierung und/oder eine Überprüfung auf Änderungen vorgenommen. Derartige Schritte bieten eine Aufbereitung, welche besonders gut die Erfordernisse einer Übertragung in ein Niedrigenergie-Hochreichweitennetz erfüllt.

In einer Ausgestaltung kann wenigstens ein Geräteparameter kumulativ gezählt werden. Beispielsweise wird die Therapiestundenanzahl kumulativ gezählt. Möglich ist auch, dass wenigstens ein Benutzerparameter kumulativ gezählt wird. Eine kumulative Zählweise bietet erhebliche Vorteile gegenüber der Verwendung von Indizes, beispielsweise einem Tagesstundenzähler. Dadurch kann auch bei einer ausbleibenden Übertragung, beispielsweise bei schlechter Netzqualität, eine wenigstens näherungsweise Schätzung der nicht übertragenen Daten vorgenommen werden. Beispielsweise kann anhand einer kumulativen Therapiestundenanzahl auf die Therapiestunden in einem Zeitraum geschlossen werden, in welchem keine Daten übermittelt werden konnten. Eine solche Ausgestaltung des Beatmungsgerätes bzw. der Datenverarbeitungseinrichtung ist daher besonders von Vorbei-, wenn die Daten über ein Niedrigenergie-Hochreichweitennetz übertragen werden.

Bevorzugt kann die Aufbereitung der Therapiedaten wenigstens eine Mittelwertbildung umfassen. Insbesondere werden die Therapiedaten einer Epoche, in der eine erwartete Empfangsbestätigung über die empfangenen Therapiedaten durch das Netz ausgeblieben ist, durch eine Aufbereitung gemittelt. Bei einer nachfolgenden Übertragung wird dann für die entsprechenden Therapiedaten vorzugsweise nur der Mittelwert übertragen. Insbesondere wird dann für einen Therapieverlauf und/oder eine Therapiestatistik nur der Mittelwert übertragen. Eine solche Aufarbeitung hat den Vorteil, dass nach einer längeren Epoche ohne verlässliche Verbindung zum Netz keine ungünstig hohe Datenmenge anfällt, welche einen hohen Energieaufwand bzw. Kostenaufwand erfordert oder die Übertragung anderer Daten blockiert.

Möglich kann auch eine andere gezielte Aufarbeitung der Therapiedaten sein, welche die Daten dieser Epoche komprimiert. Insbesondere ist eine statistische Aufarbeitung vorgesehen, um aus einer Gruppe von Werten nur einen oder eine geringere Anzahl von repräsentativen Werten zu bilden. Möglich ist auch, dass eine Gruppe von Werten durch wenigstens eine Funktion wenigstens näherungsweise beschrieben wird. Vorzugsweise wird dann die Funktion übertragen.

In allen Ausgestaltungen kann es bevorzugt sein, dass die Übertragung der Therapiedaten auch ohne eine Rückmeldung des Netzes vorgenommen wird. Insbesondere wird die Übertragung der Therapiedaten auch ohne eine Empfangsbestätigung des Netzes durchgeführt bzw. fortgesetzt. Besonders bevorzugt ist ein Betrieb des Beatmungsgerätes vorgesehen, welcher unabhängig von der Übertragung der Therapiedaten ist. Vorzugsweise ist ein Betrieb des Beatmungsgerätes auch ohne den Empfang eines Downlinks durch das Netz möglich. Dadurch ist zum einen die Nutzung der Vorteile einer netzbasierten Übertragung möglich und zum anderen wird stets ein zuverlässiger Betrieb des Beatmungsgerätes gewährleistet.

In einer vorteilhaften Ausgestaltung kann der Verbindungsaufbau der Übertragungseinrichtung zum Funknetz in einer dynamischen Taktung erfolgen. Insbesondere wird für eine Ferneinstellung des Beatmungsgerätes und/oder für eine initiale Therapiephase die Taktung gezielt erhöht. Dadurch kann sichergestellt werden, dass ein möglichst schneller Verbindungsaufbau erfolgt. Vorzugsweise wird die Taktung gezielt auf eine Maximallatenz erhöht. Insbesondere erfolgt nach dem Verbindungsaufbau die Übertragung der Therapiedaten. Die maximale Latenz wird insbesondere durch wenigstens ein Netzwerkprotokoll und/oder wenigstens eine Gerätekonfiguration der Übertragungseinrichtung vorgegeben.

Es kann möglich sein, dass die Änderung der Taktung auch durch wenigstens eine Benutzereingabe ausgelöst werden kann. Beispielsweise ist die Betätigung einer Bedieneinrichtung mit einer Erhöhung und/oder Erniedrigung der Taktung gekoppelt. Beispielsweise dann, wenn der Benutzer einen Modus auswählt, welcher eine Ferneinstellung erfordert bzw. für welchen eine Ferneinstellung hilfreich ist.

Möglich könnte auch sein, dass die Taktung in Abhängigkeit eines Therapiemodus erhöht wird, beispielsweise während eines Therapiemodus für einen kritischen Atemzustand. In anderen Betriebsmodi außerhalb der Ferneinstellung und/oder einer initialen Therapiephase ist vorzugsweise eine niedrigere Taktung vorgesehen.

Es kann auch vorgesehen sein, dass für eine Ferneinstellung und/oder für eine initiale Therapiephase wenigstens eine andere Netzverbindung als das Funknetz ausgewählt wird. Beispielsweise kann eine andere Netzverbindung ausgewählt werden, wenn auch die auf eine maximale Latenz erhöhte Taktung nicht zu einem erfolgreichen Verbindungsaufbau führt.

Besonders bevorzugt kann eine Verbindung zwischen dem Beatmungsgerät und einem Niedrigenergie-Hochreichweitennetz nur vonseiten des Beatmungsgerätes initiiert werden. Das hat den Vorteil, dass das Beatmungsgerät bzw. die Übertragungseinrichtung nicht in einem Energie verzehrenden und kostenintensiven Empfangsmodus verbleiben müssen.

Der Verbindungsaufbau der Übertragungseinrichtung zum Funknetz kann durch wenigstens einen Downlink aus wenigstens einer anderen Netzverbindung als dem Funknetz initiiert werden. Das ist von Vorteil, da Basisstationen von energieoptimierten Netzen oft keinen Verbindungsaufbau initiieren können. Daher ist für eine Initiierung der Verbindung von außerhalb des Beatmungsgerätes die andere Netzverbindung besonders hilfreich.

Beispielsweise kann durch die andere Netzverbindung ein Verbindungsaufbau initiiert werden, wenn eine Gerätekonfiguration oder eine Ferneinstellung an das Beatmungsgerät übertragen werden sollen. Die anschließende Übertragung erfolgt bevorzugt nicht mehr über die andere Netzverbindung, sondern über das Funknetz. Dadurch sind die Energie und Kostenvorteile des Niedrigenergie-Hochreichweitennetzes dennoch nutzbar.

Möglich könnte auch sein, dass der Verbindungsaufbau der Übertragungseinrichtung zum Netz durch wenigstens eine Benutzereingabe initiiert wird. Eine Initiierung des Verbindungsaufbaus durch eine Benutzereingabe kann beispielsweise dann erfolgen, wenn der Benutzer einen Anruf oder eine SMS von einem Betreuer erhalten hat. Möglich ist aber auch, dass eine Verbindung zwischen Beatmungsgerät und Niedrigenergie-Hochreichweitennetz auch durch das Niedrigenergie-Hochreichweitennetz bzw. von dessen Basisstation aus initiiert werden kann. Die Initiierung kann auch als ein Steuerbefehl in einer Gerätekonfiguration hinterlegt sein, sodass während des Betriebs des Beatmungsgeräts eine automatische Initiierung erfolgt.

Es kann in allen Ausgestaltungen bevorzugt sein, dass der Betrieb und insbesondere eine Frequenzauswahl der Übertragungseinrichtung unter Berücksichtigung wenigstens einer regionalen Vorgabe des Funknetzes erfolgt. Die Vorgabe wird vorzugsweise anhand wenigstens eines Sschrittes aus einer Gruppe von Schritten ermittelt, umfassend: wenigstens eine Trägerprüfung der empfangbaren Netze (Listen Before Talk), Empfang und Auswertung wenigstens eines GPS-Signals, Auswertung wenigstens einer GPS-Kennung wenigstens einer Basisstation des Netzes und/oder einer anderen Netzverbindung, Berücksichtigung wenigstens einer Benutzereingabe, Berücksichtigung wenigstens einer regionalspezifischen Gerätekonfiguration.

Dadurch können regionale Limitierungen besonders gut erkannt und berücksichtigt werden. Da die Anforderungen und Reglementierungen von Niedrigenergie-Hochreichweitennetzen regional oft sehr unterschiedlich sind, ist dies besonders vorteilhaft. Dadurch ist auch ein überregionaler Betrieb des Beatmungsgerätes zuverlässig möglich.

Beispielsweise kann die Übertragung von Therapiedaten unter Berücksichtigung der regionalen Vorgabe aktiviert und/oder deaktiviert werden. Möglich ist auch, dass unter Berücksichtigung der regionalen Vorgabe wenigstens eine Kenngröße für die Übertragung, wie z. B. eine Sendefrequenz, Sendeleistung, Sendezeit, Verhalten bezüglich Listen-before-Talk oder Duty Cycle festgelegt wird. Dadurch kann beispielsweise verhindert werden, dass die Übertragungseinrichtung auf nicht freigegebene Frequenzen einer bestimmten Region eingestellt wird. Insbesondere wird anhand der regionalen Vorgabe wenigstens ein Netzwerkprotokoll aus einer Gruppe von Netzwerkprotokollen ausgewählt und/oder eingestellt. Die regionalspezifische Gerätekonfiguration umfasst beispielsweise wenigstens eine Spracheinstellung und/oder wenigstens eine Auswahl einer Zeitzone und/oder wenigstens ein eine Auswahl eines Regionalschemata.

Es könnte möglich sein, dass die Therapiedaten wenigstens teilweise verschlüsselt übertragen werden. Dazu ist wenigstens ein Schlüssel vorgesehen, welcher vorzugsweise wenigstens über eine andere Netzverbindung als das Funknetz übertragen wird. Dadurch ist eine besonders sichere Übertragung der Therapiedaten möglich. Beispielsweise ist eine Ende-zu-Ende Verschlüsselung vorgesehen. Insbesondere werden die Therapiedaten gegenüber dem Netz verschlüsselt. Es ist möglich, dass das Beatmungsgerät wenigstens einen Kryptochip umfasst. Insbesondere wird der Schlüssel dynamisch aufseiten des Beatmungsgerätes und/oder auf Netzseite aus Informationen generiert, die insbesondere beiden Seiten bekannt sind.

Beispielsweise können dazu Seriennummern und/oder Identifikationsnummern und/oder Versionskennzeichnungen herangezogen werden. Möglich ist auch, dass der Schlüssel wenigstens teilweise statisch im Beatmungsgerät hinterlegt wird. Besonders bevorzugt werden mehrere Verschlüsselungsverfahren miteinander kombiniert.

Die Übertragungseinrichtung kann wenigstens eine Modemeinrichtung umfassen. Dabei wird vorzugsweise wenigstens bei einer ersten Inbetriebnahme des Beatmungsgerätes eine Erkennung der Modemeinrichtung durchgeführt. Möglich ist auch, dass wiederholt eine Erkennung der Mcdemeinrichtung durchgeführt wird. Vorzugsweise wird anhand der Erkennung ein hinterlegtes Netzwerkprotokoll ausgewählt. Möglich ist auch, dass anhand der Erkennung eine Gerätekonfiguration eingestellt wird. Beispielsweise kann die Gerätekonfiguration so eingestellt werden, dass bestimmte Betriebsarten aktiviert und/oder deaktiviert werden, welche von der Übertragung von Therapiedaten durch die Modemeinrichtung abhängig sind.

Durch eine solche Ausgestaltung der Erfindung können eine Vielzahl von Übertragungseinrichtungen bzw. Modemeinrichtungen mit einer Vielzahl von verschiedenen Typen von Beatmungsgeräten kombiniert werden. So können die Modemeinrichtungen gezielt nach den Vorgaben und Möglichkeiten von Niedrigenergie-Hochreichweitennetzen ausgewählt werden, ohne dass die Beatmungsgeräte aufwendigen Anpassungen unterzogen werden müssen. Durch die Erkennung bei der Inbetriebnahme wird das Beatmungsgerät dann so eingestellt, dass ein optimaler Betrieb mit dem jeweiligen Modemtyp möglich ist. Eine solche Ausgestaltung ist zudem besonders vorteilhaft, da gerade bei Niedrigenergie-Hochreichweitennetzen eine Vielzahl von unterschiedlichen Betreibern und regionalen Vorgaben zu beachten ist.

Die Übertragungseinrichtung kann wenigstens zeitweise über wenigstens einen Energiespeicher mit Energie versorgt werden. Der Energiespeicher wird vorzugsweise während eines Betriebs des Beatmungsgerätes aufgeladen. Insbesondere umfasst der Energiespeicher wenigstens einen Kondensator. Beispielsweise ist wenigstens ein Goldcap-Kondensator vorgesehen. Das ermöglicht eine Übertragung von Therapiedaten, welche unabhängig vom Versorgungsnetz des Beatmungsgerätes ist.

Besonders vorteilhaft kann eine solche Ausgestaltung auch dann sein, wenn das Beatmungsgerät mobil und vorzugsweise mit einem Akkubetrieb ausgestattet ist. Dann kann beispielsweise eine redundante Übertragung von Therapiedaten über den gesamten Tag erfolgen, ohne dass das Beatmungsgerät am Versorgungsnetz angebunden sein muss oder der Akku belastet wird.

Zudem kann ein Kondensator als Energiespeicher für die Übertragung in ein Niedrigenergie-Hochreichweitennetz besonders vorteilhaft sein. Derartige Netze ermöglichen eine so energieeffiziente Übertragung von Daten, dass die Energie eines entsprechenden Kondensators ausreichend ist. Zur Verwendung in einem GSM-basierten Mcbilfunknetz wäre ein solcher Energiespeicher in der Regel nicht ausreichend.

In allen Ausgestaltungen kann es besonders bevorzugt sein, dass für die Therapiedaten wenigstens ein Datensatz aus einer Gruppe von Datensätzen gespeichert wird, umfassend: erfasste und/oder eingestellte Geräteparameter, erfasste Patientenparameter, erfasste und/oder ausgewertete Therapieverläufe, registrierte und/oder ausgewertete Therapiestatistiken, Änderungen und/oder Einstellungen von Gerätekonfigurationen, Meldungen an den Patienten, Fehlerberichte.

In allen Ausgestaltungen kann es zudem besonders bevorzugt sein, dass ein Niedrigenergie-Hochreichweitennetz und vorzugsweise ein Low Power Wide Area Network (LPWAN) zur Übertragung der Therapiedaten genutzt wird. Es kann auch eine Verbindung zu wenigstens einem anderen Netz vorgesehen sein, welches energieoptimiert und/oder kostenoptimiert gegenüber üblichen Mobilfunknetzen ist. Vorzugsweise sind Netze vorgesehen, die auf einen geringen Stromverbrauch der Endgeräte, auf eine geringe Datenrate und auf eine hohe Reichweite und damit einhergehende tiefe Durchdringung auch von Bebauung optimiert sind.

Die Übertragungseinrichtung kann sich insbesondere zu wenigstens einer Basisstation eines Niedrigenergie-Hochreichweitennetzes verbinden. Über die Verbindung zum Niedrigenergie-Hochreichweitennetz können auch Daten empfangen werden, insb. als Downlink. Die Therapiedaten werden vorzugsweise von dem Niedrigenergie-Hochreichweitennetz weiter zu einer Auswerteinrichtung zur therapeutischen und/oder diagnostischen Analyse gesendet.

Mit der Übertragungseinrichtung kann auch eine Verbindung über das Netz zu wenigstens einem weiteren Beatmungsgerät aufgebaut werden. Dadurch ist vorzugsweise eine Vernetzung von zwei oder mehr Beatmungsgeräten möglich. Die Übertragungseinrichtung kann auch eine Verbindung über das Netz zu anderen Telemetrie-Einrichtungen aufbauen. Möglich sind auch Verbindungen zu anderen Sensornetzwerken und/oder zum Internet der Dinge (Internet of Things, IoT).

In einer anderen erfindungsgemäßen Ausgestaltung kann die Datenverarbeitungseinrichtung eines Beatmungsgerätes und insbesondere eines Heimbeatmungsgerätes und/oder Schlaftherapiegerätes dienen. Anhand der Datenverarbeitungseinrichtung werden Therapiedaten registriert und in wenigstens einer Speichereinrichtung der Datenverarbeitungseinrichtung hinterlegt. Wenigstens ein Teil der hinterlegten Therapiedaten wird mit wenigstens einer Übertragungseinrichtung an wenigstens ein Netzwerk übertragen. Dabei wird mit der Übertragungseinrichtung wenigstens eine Funkverbindung zu wenigstens einem Netz aufgebaut. Mit der Datenverarbeitungseinrichtung wird wenigstens eine Aufbereitung der Therapiedaten vor ihrer Übertragung in das Netz vorgenommen.

Dieseerfindungsgemäße Datenverarbeitungseinrichtung bietet ebenfalls viele Vorteile, da die erfindungsgemäße Aufbereitung der Therapiedaten eine zuverlässige Datenübertragung auch an Netze mit einer eingeschränkten Verfügbarkeit bzw. Übertragungsqualität ermöglicht. Dadurch können zur Übertragung auch Niedrigenergie-Hochreichweitennetze und z. B. ein Low Power Wide Area Network (LPWAN) herangezogen werden.

Das erfindungsgemäße Beatmungsgerät umfasst wenigstens eine Beatmungseinrichtung zur Erzeugung eines Luftstroms für eine Atemtherapie. Anhand wenigstens einer Datenverarbeitungseinrichtung sind Therapiedaten registrierbar. Die Therapiedaten sind in wenigstens einer Speichereinrichtung hinterlegbar. Wenigstens eine Übertragungseinrichtung ist dazu geeignet und ausgebildet, wenigstens einen Teil der hinterlegten Therapiedaten an ein Netzwerk zu übertragen. Dabei ist die Übertragungseinrichtung dazu geeignet und ausgebildet, wenigstens ein verfügbares Netz zu ermitteln und wenigstens eine Kenngröße für eine Netzqualität des Niedrigenergie-Hochreichweitennetzes zu bestimmen. Die Datenverarbeitungseinrichtung ist zudem dazu geeignet und ausgebildet, eine Aufbereitung der Therapiedaten vor ihrer Übertragung in das Netz in Abhängigkeit der Kenngröße dynamisch vorzunehmen.

Auch das erfindungsgemäße Beatmungsgerät hat viele Vorteile. Ein besonderer Vorteil kann die Datenverarbeitungseinrichtung sein, sodass die Therapiedaten vor ihrer Übertragung in das Netz in Abhängigkeit der Kenngröße dynamisch aufbereitet werden können. Dadurch kann das Beatmungsgerät beispielsweise zur Übertragung von Therapiedaten in ein Niedrigenergie-Hochreichweitennetz eingesetzt werden.

Vorzugsweise ist Beatmungsgerät dazu geeignet und ausgebildet, in der erfindungsgemäßen Weise betrieben zu werden. Die Übertragungseinrichtung ist insbesondere dazu geeignet und ausgebildet, sich mit wenigstens einem Niedrigenergie-Hochreichweitennetz und vorzugsweise einem Low Power Wide Area Network (LPWAN) zu verbinden. Zur Erfassung der Therapiedaten umfasst das Beatmungsgerät vorzugsweise wenigstens eine Überwachungseinrichtung und/oder wenigstens eine Sensoreinrichtung.

Weitere Vorteile und Merkmale der vorliegenden Erfindung ergeben sich aus der Beschreibung der Ausführungsbeispiele, welche mit Bezug auf die beiliegenden Figuren im Folgenden erläutert werden.

In den Figuren zeigen:
- Fig. 1: eine schematische Darstellung eines erfindungsgemäßen Beatmungsgerätes in einer perspektivischen Ansicht; und
- Fig. 2: eine stark schematische Darstellung des Beatmungsgerätes.

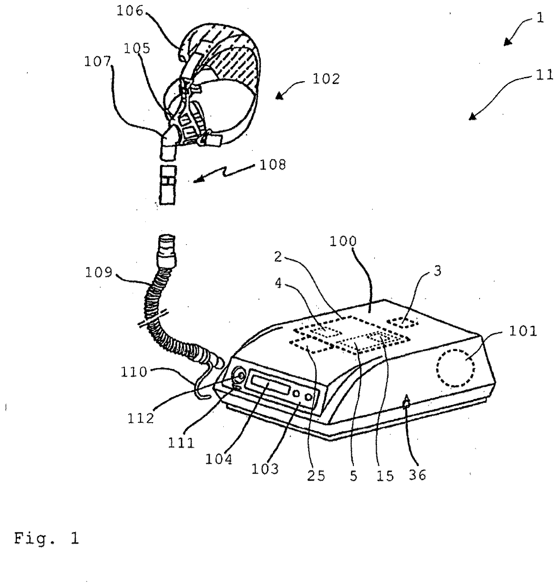

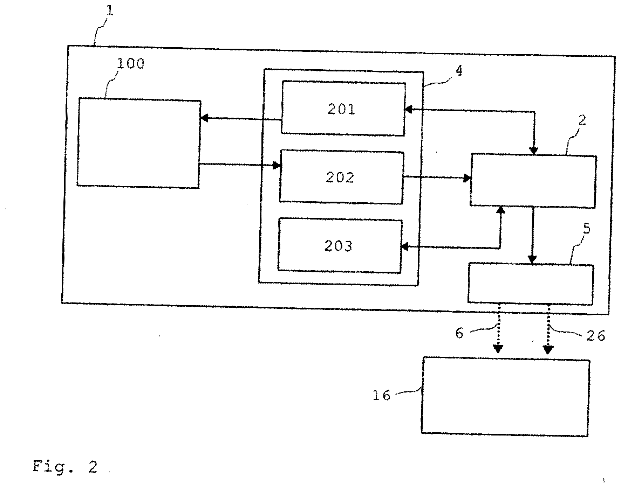

Die Figuren 1 und 2 zeigen ein Beatmungsgerät 1, welches als ein Heimbeatmungsgerät 11 bzw. Schlaftherapiegerät ausgebildet ist. Das Beatmungsgerät 1 kann aber auch als klinisches Beatmungsgerät 1 eingesetzt werden. Das Beatmungsgerät 1 ist zur erfindungsgemäßen Datenverarbeitung mit der Datenverarbeitungseinrichtunggeeignet und ausgebildet.

Das Beatmungsgerät - umfasst eine Beatmungseinrichtung 100 mit einer Gebläseeinrichtung 101 zur Erzeugung eines Luftstromes für die Beatmung. Zur Steuerung der Beatmungseinrichtung 100 und zur Erfassung Therapiedaten ist hier eine Überwachungseinrichtung 3 vorgesehen. Die Bedienung und Einstellung des Beatmungsgerätes 1 erfolgt über eine Bedieneinrichtung 103 mit einer Anzeigeeinrichtung 104.

Das Beatmungsgerät 1 weist eine Atemschnittstelle 102 auf, um den Luftstrom einem Benutzer zur Beatmung zuzuführen. Die Atemschnittstelle 102 ist vorzugsweise als ein Patienteninterface ausgebildet und kann beispielsweise als eine Vollgesichtsmaske, als ein Nasal-Pillow, als ein Tubus oder als eine Larynxmaske ausgestaltet sein. Die hier gezeigte Atemschnittstelle 102 ist eine als Nasalmaske ausgebildete Atemmaske 105. Zur Fixierung der Atemmaske 105 ist eine Kopfhaube 106 vorgesehen.

Zur Verbindung der Atemschnittstelle 102 mit der Beatmungseinrichtung 100 ist ein Verbindungsschlauch 109 vorgesehen, der mittels einer Koppeleinrichtung 112 mit der Beatmungseinrichtung 100 verbunden wird. Über ein Kuppelungselement 107 wird der Beatmungsschlauch 109 mit der Atemschnittstelle 102 verbunden. Zwischen dem Beatmungsschlauch 109 und dem Kuppelungselement 107 ist ein Ausatmungselement 108 angeordnet, welches ein Ventil umfasst oder als ein solches ausgebildet ist. Das Ausatmungselement 108 ist insbesondere dazu vorgesehen, während der Ausatmung des Benutzers ein Rückatmen in das Beatmungsgerät 1 zu verhindern.

Die Überwachungseinrichtung 3 ist hier mit einer nicht näher dargestellten Senscreinrichtung wirkverbunden, welche einen oder mehrere Sensoren zur Erfassung von Geräteparametern 201 und/oder Patientenparametern und/oder anderer für die Beatmung charakteristischer Größen aufweist.

Zum Beispiel umfasst die Überwachungseinrichtung 3 einen hier nicht näher gezeigten Drucksensor, welcher die Druckverhältnisse bezüglich der Atemschnittstelle 102 erfasst. Dazu ist der Drucksensor über einen Druckmessschlauch 110 mit der Atemschnittstelle 102 verbunden. Über einen Eingangsstutzen 111 ist der Druckmessschlauch 110 an die Überwachungseinrichtung 3 angebunden.

Des Weiteren dient die Überwachungseinrichtung 3 hier zur Ansteuerung der Gebläseeinrichtung 101 auf. Die Überwachungseinrichtung 3 stellt einen notwendigen Minimaldruck bereit und kompensiert Druckschwankungen, die durch die Atemtätigkeit ces Benutzers bedingt sind. Beispielsweise erfasst die Überwachungseinrichtung 3 auch den gegenwärtigen Druck in der Atemmaske 105 und regelt die Leistung der Gebläseeinrichtung 101 entsprechend nach, bis ein gewünschter Beatmungsdruck anliegt. Die zur Einstellung der Beatmungseinrichtung 100 bzw. der Gebläseeinrichtung 101 benötigten Geräteparameter 201 sowie die Gerätekonfiguration und/oder Gerätesoftware sind in einer Speichereinrichtung 4 hinterlegt.

Die Überwachungseinrichtung 3 kann hier auch zur Erfassung von Patientenparametern ausgebildet sein. Die Überwachungseinrichtung 3 kann dazu mit Sensoren zur Messung der Atemexkursion, zur Messung einer Sauerstoffsättigung des Blutes und/oder zur Messung einer EEG-, einer EMG-, einer EOG- oder einer EKG-Aktivität ausgestattet sein.

Das hier gezeigte Beatmungsgerät 1 kann als ein Fix-Level-Gerät oder auch als ein Automatic-Level-Gerät ausgebi_det sein. Insbesondere erfolgt dabei durch die Überwachungseinrichtung 3 eine Regelung auf Soll-Geräteparameter 201, welche zuvor anhand der charakteristischen Atmung eines Benutzers individuell berechnet und festgelegt worden sind.

Es ist auch möglich, das die Beatmungseinrichtung 100 dynamisch und insbesondere je nach Atemphase des Benutzers angepasst wird. Beispielsweise kann anhand der Überwachungseinrichtung 3 ein Atemphasenwechsel erkannt werden, sodass je nach Atemphase ein höherer bzw. niedrigerer Druck bereitgestellt werden kann. Beispielsweise kann das Beatmungsgerät 1 als ein CPAP- oder APAP-Gerät ausgebildet sein. Das Beatmungsgerät 1 kann auch als ein Bilevel-Gerät ausgebildet sein. Beispielsweise reagiert das Beatmungsgerät 1 auf bestimmte Atemereignisse, wie z. B. Schnarchen, Atemabflachungen und/cder obstruktive Druckspitzen mit entsprechenden Einstellungen der Geräteparameter 201.

Die von der Überwachungseinrichtung 3 erfassten Druckverhältnisse werden zusammen mit weiteren Geräteparametern 201 in einer Speichereinrichtung 4 hinterlegt. Zudem werden die von der Überwachungseinrichtung 3 eingestellten Druckverhältnisse bzw. die vorgenommenen Druckanpassungen ebenfalls als Geräteparameter 201 in der Speichereinrichtung 4 hinterlegt. Auch die erfassten Patientenparameter können in der der Speichereinrichtung 4 hinterlegt werden.

Als Geräteparameter 201 können beispielsweise ein Start-Therapie-Druck, ein maximaler Therapie-Druck, ein minimaler Therapie-Druck und/oder ein Zielvolumen und/oder andere zur Einstellung der Beatmungseinrichtung 100 geeignete Geräteparameter 201 hinterlegt sein. Diese Geräteparameter 201 werden von der Überwachungseinrichtung 3 zur Einstellung der Beatmungseinrichtung 100 aus der Speichereinrichtung 4 abgerufen.

Des Weiteren werden die über den Therapiezeitraum erfassten Druckverhältnisse und/oder sonstigen Geräteparameter und/oder Patientenparameter im Rahmen von Therapieverläufen 202 in der Speichereinrichtung 4 hinterlegt. Als Therapieverläufe 202 können z. B. ein Flow-Verlauf, ein Druck-Verlauf und/oder ein Event-Verlauf registriert werden. Die Therapieverläufe 202 werden der Speichereinrichtung 4 vorzugsweise von der Überwachungseinrichtung 3 bereitgestellt, die diese Daten während der Therapie erfasst.

Die in der Speichereinrichtung 4 hinterlegten Geräteparameter 201 und/oder Patientenparameter und/oder Therapieverläufe werden hier von einer Datenverarbeitungseinrichtung 2 abgerufen und zu einer oder mehrerer Therapiestatistiken 203 ausgewertet. Die Therapiestatistik 203 wird wiederum in der Speichereinrichtung 4 hinterlegt. Die Therapiestatistik 203 kann z. B. als Statistik für jeweils eine einzelne Therapiebehandlung ausgestaltet sein. Dabei können z. B. ein mittlerer Druck und/oder die Therapiedauer und/oder ein Leckageparameter ermittelt und abgespeichert werden.

Die Therapiestatistik 203 kann auch als Statistik für jeweils eine Therapieperiode ausgestaltet sein, welche mehrere Therapiebehandlungen umfasst. Dabei können z. B. gemittelte Werte für den mittleren Druck und/oder die Therapiedauer und/oder den Leckageparameter ermittelt und abgespeichert werden. Die Therapiestatistik 203 kann auch eine Auswertung der Mitwirkungsbereitschaft des Benutzers umfassen.

Die wie zuvor beschrieben in der Speichereinrichtung 4 abgelegten Werte und Größen werden im Rahmen der vorliegenden Erfindung als Therapiedaten bezeichnet. Um die Therapiedaten einer therapeutischen bzw. diagnostischen Analyse unterziehen zu können oder um die Funktion des Beatmungsgerätes 1 überwachen zu können, ist hier eine Übertragung der Therapiedaten an ein Netzwerk 16 z. B. über das Internet vorgesehen. Das Netzwerk umfasst vorzugsweise wenigstens einen Server.

Über das Netzwerk 16 kann ein Zugriff auf die Therapiedaten von einem entfernten Ort erfolgen, sodass eine crtsunabhängige Auswertung möglich ist. Die Übertragung erfolgt mittels einer Übertragungseinrichtung 5 drahtlos bzw. per Funkverbindung an ein Funknetz 6. Dazu umfasst die Übertragungseinrichtung 5 hier eine Modemeinrichtung 15.

Ergänzend zu der Übertragung über das Funknetz 6 können die Therapiedaten auch wenigstens teilweise über eine drahtlose und/oder drahtgebundene alternative Netzverbindung 26 gesendet werden. Zudem können die Therapiedaten auch wenigstens teilweise auf einem Speichermedium 36 abgelegt werden. Das Speichermedium 36 ist beispielsweise als eine Speicherkarte oder eine Festplatte oder ein USB-Massenspeicher ausgebildet.

Um eine vorübergehend vom Stromnetz unabhängige Energieversorgung der Übertragungseinrichtung 5 zu ermöglichen, ist hier ein Energiespeicher 25 vorgesehen. Der Energiespeicher 25 wird vorzugsweise während eines Betriebs im Stromnetz aufgeladen. Der Energiespeicher 25 umfasst hier einen oder mehrere Kondensatoren und beispielsweise Goldcap-Kondensatoren. Beispielsweise ist eine mehrstündige oder mehrtägige Energieversorgung der Übertragungseinrichtung 5 vorgesehen.

Der Energiespeicher 25 ermöglicht eine besonders effektive Nutzung der Low Power-Eigenschaften von Niedrigenergie-Hochreichweitennetzen. So kann mit einem Goldcap-Kondensator die Datenübertragung über einen Tag gestreckt werden, auch wenn das Gerät 1 von der Stromversorgung genommen wird. Dafür kann entweder die komplette Protokolllogik im Modem 15 implementiert sein. Oder es werden Sende-Queue und Empfangspuffer implementiert, sodass Modems 15 generisch für viele Geräte 1 eingesetzt werden können.

Die Übertragungseinrichtung 5 ermittelt hier wenigstens eine Kenngröße 7 für eine Netzqualität des verfügbaren Funknetzes 6. Als Netzqualität wird beispielsweise eine Verfügbarkeit und/oder eine Übertragungszuverlässigkeit des Funknetzes 6 ermittelt. Dazu kann z. B. eine Signalstärke des Funknetzes 6 herangezogen werden. Die Datenverarbeitungseinrichtung 2 nimmt daraufhin eine Aufbereitung der Therapiedaten vor, welche je nach ermittelter Kenngröße 7 der Netzqualität gezielt angepasst wird. Die Datenverarbeitungseinrichtung 2 kann auch eine vorbestimmte Aufbereitung der Therapiedaten vornehmen, sodass auf eine Ermittlung der Kenngröße 7 verzichtet werden kann.

Um die Übertragungskosten für die Therapiedaten möglichst gering zu halten oder einzusparen, wird hier als Funknetz 6 bevorzugt ein Niedrigenergie-Hochreichweitennetz und besonders bevorzugt ein Low Power Wide Area Network (LPWAN) herangezogen. Die Übertragungskosten pro Endstelle und Jahr liegen vorteilhafterweise bei einem Bruchteil derer von GSM-oasierten Netzen.

Möglich sind Funknetze 6, die in lizenzierten Frequenzbereichen und/oder lizenzfreien Frequenzbereichen kommunizieren. Beispielsweise kann LTE-M vorgesehen sein, welches eine gegenüber 3G LTE deutlich schmalere Bandbreite von 200 kHz und vereinfachte Protokolle und Modulationsschema aufweist, die weniger Anforderungen an Rechenkapazität stellen.

Die Übertragung in lizenzfreien Bändern erfolgt insbesondere um 868 MHz (Europa) und um 905-915 MHz (USA) oder gemäß den lokalen Regularien in anderen Staaten. Die Datenübertragungsrate beträgt z. B. <100 bit/s (Uplink, Europa). Möglich ist auch eine Datenrate zwischen 300 bit/s bis 50 kbit/s. Das Datenvolumen beschränkt sich z. B. auf 140 Nachrichten von 12 Byte Payload vom Gerät zu Server (Uplink) und insbesondere 4 Nachtrichten von 8 Byte Payload in Gegenrichtung (Downlink). Der Downlink kann vorzugsweise nur auf Initiative der Endstelle selbst erfolgen, d. h. eine Verbindung kann nicht vom Server initiiert werden. Möglich sind jedoch auch Übertragungsklassen mit zeitgetakteten Downlink-Phasen, d. h. mit garantierter Maximallatenz, sowie mit permanenter Downlinkmöglichkeit definiert, sodass eine permanente bidirektionale Verbindung möglich ist.

Die Reichweite beträgt z. B. 3-10 km in städtischen und 30-50 km in ländlichen Gebieten sowie > 1000 km bei Sichtverbindung.

Die übertragenen Daten stehen vorzugsweise auf zentralen Servern zum Abruf bereit. Über diese Server können auch Daten für den Downlink bereitgestellt werden.

Die hier vorgestellte Datenverarbeitungseinrichtung hat den besonderen Vorteil, dass bei der Übertragung der Therapiedaten LPWAN-Netze 6 eingesetzt werden können. Durch die dynamische Datenaufarbeitung je nach Netzqualität des Funknetzes 6 kann so eine zuverlässige Übertragung der Therapiedaten ohne relevante Verluste auch über LPWAN-Netze 6 erreicht werden.

Eine Aufarbeitung der Therapiedaten für ihre Übertragung im LPWAN-Netz 6 ist nachfolgend anhand von Beispielen näher beschrieben.

Das im LPWAN-Netz 6 übertragbare Datenvolumen ist in der Regel streng limitiert. Daher umfasst die Aufarbeitung der Therapiedaten bevorzugt eine Datenkomprimierung. Insbesondere wird jedes Bit genutzt und z. B. eine Bitmaske angewendet. Die Wertebereiche der Therapiedaten werden insbesondere maximal beschränkt. Beispielsweise für die Übertragung von Einstellmöglichkeiten für einen Softstart zwischen 0 min und 45 min sind in 5min-Schritten nur 4 Bit für die 10 Stufen vorgesehen.

Die Therapiedaten werden bevorzugt priorisiert, sodass nur entsprechend wichtige Therapiedaten übertragen werden. Dabei erfolgt ein Abgleich der zugewiesenen Prioritäten im Rahmen von Schwellenwerten. Die Zuordnung der Prioritäten erfolgt insbesondere nach der Wichtigkeit der Daten und bevorzugt auch unter Berücksichtigung der Kenngröße für die Netzqualität.

Es werden insbesondere Differenzen statt Absolutwerten übertragen. Werte oder Datenblöcke werden insbesondere nur bei Änderungen übertragen. Beispielsweise werden Geräteeinstellungen bzw. Gerätekonfigurationen nur übertragen, wenn sie sich geändert haben.

Die Beschränkungen des Datenvolumens gelten in der Regel auch für Basisstationen bzw. Gateways, da die zur Verfügung stehende Downlink-Zeit von allen an einem Gateway angebundenen Endgeräten geteilt werden muss. Dadurch werden vorzugsweise auch Downlinks aufgearbeitet und insbesondere limitiert.

Beispielsweise werden Empfangsbestätigungen bzw. Sendungsbestätigungen nur in definierten Abständen verschickt oder nur, wenn auch Nutzdaten wie z. B. neue Geräteeinstellungen für das Beatmungsgerät 1 vorliegen. Datenübertragungen, die ein größeres Datenvolumen erfordern, werden insbesondere auf Parallelkanäle und bevorzugt auf die andere Netzverbindung 26 ausgelagert. Zum Beispiel erfolgt ein Detaildatenupload per Uploadprogramm auf dem PC oder Smart Device des Benutzers oder über ein GSM-basiertes Modem oder per Einsenden eines Speichermediums 36. Die Modemeinrichtung 15 ist vorzugsweise auch zur GSM-basierten Übertragung ausgebildet. Auch ein Firmware-Update kann per Zusenden einer SD-Karte statt über Datenfunk erfolgen.

Eine Übertragung der Therapiedaten im LPWAN-Netz 6 kann durch andere Anwendungen, die das Frequenzband benutzen, nahezu jederzeit gestört werden. Daher umfasst die Aufarbeitung der Therapiedaten einen Schutz vor Datenverlust.

Die Therapiedaten werden vorzugweise redundant übertragen. Die Anzahl der Wiederholungen wird insbesondere nach der Wichtigkeit der Daten und bevorzugt auch unter Berücksichtigung der Kenngröße für die Netzqualität festgelegt. Beispielsweise werden die Therapiedaten mehrfach und auch zu verschiedenen Tageszeiten und gegebenenfalls während Therapie versendet.

Bevorzugt werden kumulative Zähler statt oder zusätzlich zu Indizes verwendet. Insbesondere werden Betriebsstundenzähler statt der Zählung einer täglichen Nutzung verwendet. Es werden insbesondere Mittelwerte für Epochen übertragen, für die die Datenübertragung nicht bestätigt wurde. Wenn z. B. über eine Woche oder mehr kein Downlink erhalten wurde, werden Statistik-Mittelwerte für diese Woche übertragen, wenn ein Downlink wieder verfügbar ist.

Besonders wichtige Therapiedaten werden bevorzugt mit hoher Redundanz übertragen, unwichtigere mit geringerer. Es ist insbesondere eine Nachforderung von Daten vorgesehen. Insbesondere werden Checksums und Hashs verwendet.

Es wird insbesondere eine dynamische Abhängigkeit zwischen der Redundanz und dem Umfang der zu übertragenden Daten sowie der Anzahl der Übertragungen eingestellt. Dadurch kann besonders kosteneffizient übertragen werden. Dabei wird insbesondere auch die Kenngröße für die Netzqualität berücksichtigt. Wenn das Netz z. B. schwach ist und/oder gar keine Antwort kommt, werden bevorzugt Basisdatensätze redundant übertragen, z. B. Therapiestundenzähler, Basisstatistik des aktuellen Therapietages oder des Vortages. Wenn das Netz sicher überträgt, wird die Redundanz zurückgenommen und der Datenumfang kann ausgeweitet werden.

Insbesondere erfolgt eine Abwägung zwischen Kosten und Datenübertragungssicherheit. So kann das LPWAN 6 in Kombination mit der anderen Netzverbindung 26 und z. B. mit klassischem Mobilfunk verwendet werden. Die andere Netzverbindung 26 kommt vorzugsweise nur zum Einsatz, wenn das LPWAN 6 nicht ausreicht oder Daten mit erhöhten Volumen- oder Sicherheitsansprüchen übertragen werden.

Die Netzabdeckung wird bei der Anpassung des Beatmungsgerätes 1 vorzugsweise berücksichtigt. So wird z. B. eine Ferneinstellung gezielt blockiert, wenn der Downlink häufig oder immer das Gerät 1 nicht erreicht. Es kann ein Verfügbarkeits-Check durchgeführt werden. Beispielsweise auf Basis der Netzabdeckung. Dabei kann ein Einsatzort des Beatmungsgeräts 1 eingegeben werden und/oder eine zu erwartende Verbindungsqualität. Daraufhin basierend können dann nutzbare Dienste ausgegeben und z. B. die Gerätekonfiguration angepasst werden.

Im Funknetz 6 ist in der Regel eine Kenngröße 7 für eine Übertragungsqualität für einen Uplink hinterlegt. Dem Beatmungsgerät 1 wird die Kenngröße 7 daher bevorzugt im Downlink übertragen. In Abhängigkeit der mitgeteilten Kenngröße kann der Datenversand dann geräteseitig angepasst werden. Z. B. wird die Redundanz erhöht oder die Redundanz wird verringert und der Nutzdatenumfang erhöht.

Die Aufarbeitung und/oder Übertragung der Therapiedaten berücksichtigt hier, dass im LPWAN-Netz 6 eine asymmetrische Übertragung auftreten kann. Dabei funktioniert z. B. der Uplink, der Downlink aber nicht. Bei der hier vorgestellten Datenverarbeitungseinrichtung werden daher Protokolle verwendet, die auch ohne Rückmeldung vom Server Daten übertragen. Dabei wird eine gezielte Abwägung zwischen Netzbelegung und Anzahl der Nachrichten und der Redundanz getroffen.

Beispielsweise werden bestimmte Therapiedaten mit der höchsten Priorität n-fach wiederholt, bis zur Versendung weiterer Therapiedaten übergegangen wird. Da maximale Uplink-Qualität durch Erhöhen der Sendeleistung bis zur Grenze der erlaubten Abstrahlung auch bei schlechter Antenne möglich ist, werden vorzugsweise Anwendung und Beatmungsgerät 1 so ausgelegt, dass auf den Downlink bewusst komplett verzichtet wird. Das bietet erhebliche Kostenvorteile.

Vorzugweise ist eine endgeräteinduzierte Verbindung vorgesehen. Wenn eine garantierte Maximallatenz notwendig ist, d. h. das Gerät 1 spätestens innerhalb einer definierten Zeit sich mit dem Funknetz 6 verbinden soll, z. B. für eine Ferneinstellung bzw. Tele-Setting, werden vorzugsweise die nachfolgenden Schritte durchgeführt. Insbesondere wird der Verbindungsaufbau auf Maximallatenz getaktet, d. h., das Gerät 1 meldet sich in definiertem Zeitabstand beim Funknetz 6. Diese Taktung kann auch dynamisch veränderlich sein, beispielsweise lässt sich eine Maximallatenz zu den üblichen Bürozeiten der Leistungserbringer garantieren, oder in einer initialen Therapiephase, und außerhalb ist die Taktung nicht vorhanden/geringer.

Die Verbindung kann auch über die andere Netzverbindung 26 bzw. durch einen zweiten Kanal initiiert werden. Beispielsweise anhand einer SMS zum Gerät 1. Dazu ist bevorzugt eine kombinierte Modemeinrichtung 15 für GSM 26 und LPWAN 6 vorgesehen. Es kann auch ein Bedienelement am Gerät 1 zum Verbindungsaufbau vorgesehen sein, das der Benutzer z. B. auf Anruf hin drücken kann.

Bei der Übertragung der Therapiedaten erfolgt vorzugsweise eine Auswahl des regional passenden und erlaubten Frequenzbereichs sowie eine Berücksichtigung weiterer regionaler Regularien.

Eine länderspezifische Parametrierung ist vorzugsweise umschaltbar und/oder durch regional-spezifische Modems auswählbar. Es kann eine automatische Umschaltung oder Aktivierung oder Deaktivierung der Funkmodule über verschiedene Mechanismen erfolgen. Insbesondere erfolgt eine Ermittlung regionaler Vorgabe des Funknetzes 6.

Beispielsweise wird eine Trägerprüfung (Listen Before Talk) durchgeführt. Es kann auch ermittelt werden, ob es Hinweise auf LPWAN-Netze 6 im Funkäther gibt, z. B. anhand von Beacons. Es kann auch ein GPS-Signal eines eingebauten Empfängers verwendet werden. Es kann auch die GPS-Kennung der Basis-Station verwenden werden, wenn wenigstens eines der Netze 6, 26 empfangen werden. Es kann auch eine Nutzereingabe verwendet werden, die die Einsatzregion definiert. Zur Ermittlung der Einsatzregion können auch Einstellungen des Beatmungsgerätes 1 verwendet werden, wie z. B. Sprache, Regionalschema, Zeitzone.

Die Aufarbeitung der Therapiedaten umfasst insbesondere eine Verschlüsselung und Authentifizierung, z. B. AES, RSA. Beispielsweise ist eine Ende-zu-Ende-Verschlüsselung vorgesehen, damit Daten gegenüber dem Funknetz 6 verschlüsselt sind. Der Schlüssel wird entweder dynamisch auf Geräte- und Serverseite generiert oder aus Informationen, die beiden Seiten bekannt sind oder statisch im Gerät 1 hinterlegt.

Beispielsweise können dazu Serien- oder andere Identifikationsnummern, Versionskennzeichnungen herangezogen werden. Insbesondere werden mehrere Verfahren zur Verschlüsselung miteinander kombiniert. Für sensible Informationen kann ein Kryptochip verwendet werden. Die Information, aus der der Schlüssel generiert wird, werden insbesondere nicht oder zumindest nicht regelmäßig über das Funknetz 6 übertragen.

Die auf LPWAN basierenden Netze 6 stellen in der Regel ein sehr heterogenes System dar, in welchem viele Provider und viele unterschiedliche Komponenten vorherrschen. Bei einer ersten Inbetriebnahme des Beatmungsgerätes 1 wird daher eine Erkennung der Mooemeinrichtung 15 durchgeführt und anhand der Erkennung ein hinterlegtes Netzwerkprotokoll ausgewählt und/oder eine Gerätekonfiguration eingestellt. So kann trotz der Anforderungsvielfalt der Herstellungsaufwand günstig gehalten werden.

Zudem werden vorzugsweise Webschnittstellen zu verschiedenen Providern und/oder Anpassungsmodule erstellt. Die Business- und Applikationslogik hinter diesen Modulen ist für die relevanten Hersteller möglichst gleich. So wird eine Logistikinfrastruktur erreicht, die Identifizierung, Aktivierung, Deaktivierung und Zuordnung von Modems 15 und Geräten 1 in der Produktion, in der Lieferlogistik und im Betrieb für die verschiedenen Funknetze 6 ermöglicht. Beispielsweise fragt das Beatmungsgerät 1 das Modem 15 ab und wählt Verhalten und Protokoll gemäß Modemtyp.

### Bezugszeichenliste:

- 1: Beatmungsgerät
- 2: Datenverarbeitungseinrichtung
- 3: Überwachungseinrichtung
- 4: Speichereinrichtung
- 5: Übertragungseinrichtung
- 6: Funknetz
- 7: Kenngröße
- 11: Heimbeatmungsgerät
- 15: Modemeinrichtung
- 16: Netzwerk
- 25: Energiespeicher
- 26: Netzverbindung
- 36: Speichermedium
- 100: Beatmungseinrichtung
- 101: Gebläseeinrichtung
- 102: Atemschnittstelle
- 103: Bedieneinrichtung
- 104: Anzeigeeinrichtung
- 105: Atemmaske
- 106: Kopfhaube
- 107: Kupplungselement
- 108: Ausatmungselement
- 109: Beatmungsschlauch
- 110: Druckmessschlauch
- 111: Eingangsstutzen
- 112: Koppeleinrichtung
- 201: Geräteparameter
- 202: Therapieverlauf
- 203: Therapiestatistik

## Patentansprüche

1. Verfahren zum Betreiben einer Datenverarbeitungseinrichtung (2) eines Beatmungsgerätes (1), wobei Therapiedaten registriert und in wenigstens einer Speichereinrichtung (4) der Datenverarbeitungseinrichtung (2) hinterlegt werden und wobei wenigstens ein Teil der hinterlegten Therapiedaten mit wenigstens einer Übertragungseinrichtung (5) an wenigstens ein Netzwerk (16) übertragen wird,
**dadurch gekennzeichnet,**
**dass** mit der Übertragungseinrichtung (5) wenigstens ein verfügbares Funknetz (6) ermittelt und wenigstens eine Kenngröße (7) für eine Netzqualität des Funknetzes (6) bestimmt wird und dass mit der Datenverarbeitungseinrichtung (2) wenigstens eine Aufbereitung der Therapiedaten vor ihrer Übertragung in das Funknetz (6) in Abhängigkeit der Kenngröße (7) dynamisch vorgenommen wird, wobei die Therapiedaten zur Aufbereitung priorisiert werden und wobei vorrangig und/oder ausschließlich die Therapiedaten übertragen werden, welche wenigstens einen Schwellenwert für eine Priorität überschreiten.

2. Verfahren nach dem vorhergehenden Anspruch, wobei die Kenngröße eine Verfügbarkeit und/oder eine Übertragungszuverlässigkeit des Funknetzes (6) definiert.

3. Verfahren nach dem vorhergehenden Anspruch, wobei der Schwellenwert für die Priorität in Abhängigkeit der Kenngröße (7) dynamisch festgelegt wird.

4. Verfahren nach einem der beiden vorhergehenden Ansprüche, wobei einer Therapiestundenanzahl und/oder einem Therapieverlauf (202) und/oder einer Therapiestatistik (203) eine Priorität oberhalb des Schwellenwertes zugeordnet wird.

5. Verfahren nach einem der vorhergehenden Ansprüche, wobei die Therapiedaten mit einer Redundanz übertragen werden, welche in Abhängigkeit der Kenngröße (7) dynamisch eingestellt wird.

6. Verfahren nach dem vorhergehenden Anspruch und nach einem der Ansprüche 3 bis 5, wobei Therapiedaten, welche den Schwellenwert für die Priorität überschreiten, mit einer höheren Redundanz als Therapiedaten unterhalb des Schwellenwerts übertragen werden.

7. Verfahren nach einem der vorhergehenden Ansprüche, wobei in Abhängigkeit der Kenngröße (7) festgelegt wird, dass die Therapiedaten über wenigstens eine andere Netzverbindung (26) als das Funknetz (6) übertragen werden.

8. Verfahren nach einem der vorhergehenden Ansprüche, wobei in Abhängigkeit der Kenngröße (7) wenigstens eine Ferneinstellbarkeit des Beatmungsgerätes (1) eingeschränkt und/oder erweitert wird.

9. Verfahren nach einem der vorhergehenden Ansprüche, wobei zur Aufbereitung die Therapiedaten auf Änderungen überprüft werden und wobei vorrangig und/oder ausschließlich geänderte Therapiedaten übertragen werden.

10. Verfahren nach einem der vorhergehenden Ansprüche, wobei wenigstens ein Geräteparameter (201) und insbesondere die Therapiestundenanzahl kumulativ gezählt und übertragen wird.

11. Verfahren nach einem der vorhergehenden Ansprüche, wobei die Aufbereitung der Therapiedaten einer Epoche, in der eine erwartete Empfangsbestätigung über die empfangenen Therapiedaten durch das Funknetz (6) ausgeblieben ist, eine Mittelwertbildung umfasst und wobei für wenigstens einen Teil der Therapiedaten und insbesondere für einen Therapieverlauf (202) und/oder eine Therapiestatistik (203) nur der Mittelwert übertragen wird.

12. Verfahren nach einem der vorhergehenden Ansprüche, wobei der Verbindungsaufbau der Übertragungseinrichtung (5) zum Netz (6) in einer dynamischen Taktung erfolgt und wobei für eine Ferneinstellung des Beatmungsgerätes (1) und/oder für eine initiale Therapiephase die Taktung gezielt erhöht wird.

13. Verfahren nach einem der vorhergehenden Ansprüche, wobei der Verbindungsaufbau der Übertragungseinrichtung (5) zum Funknetz (6) durch wenigstens einen Downlink an das Beatmungsgerät (1) aus einer anderen Netzverbindung (26) als dem Funknetz (6) initiiert wird und/oder durch wenigstens eine Benutzereingabe initiiert wird.

14. Verfahren nach einem der vorhergehenden Ansprüche, wobei der Betrieb und insbesondere eine Frequenzauswahl der Übertragungseinrichtung (5) unter Berücksichtigung wenigstens einer regionalen Vorgabe des Funknetzes (6) erfolgt und wobei die Vorgabe anhand wenigstens eines Verfahrensschrittes aus einer Gruppe von Verfahrensschritten ermittelt wird, umfassend: wenigstens eine Trägerprüfung (Listen Before Talk), Empfang und Auswertung wenigstens eines GPS-Signals, Auswertung wenigstens einer GPS-Kennung wenigstens einer Basisstation, Berücksichtigung wenigstens einer Benutzereingabe, Berücksichtigung wenigstens einer regional-spezifischen Gerätekonfiguration.

15. Beatmungsgerät (1) mit wenigstens einer Beatmungseinrichtung (100) zur Erzeugung eines Luftstroms für eine Atemtherapie, wenigstens einer Datenverarbeitungseinrichtung (2), wenigstens einer Speichereinrichtung (4), und wenigstens einer Übertragungseinrichtung (5), wobei anhand der wenigstens einer Datenverarbeitungseinrichtung (2) Therapiedaten registrierbar und in der wenigstens einer Speichereinrichtung (4) hinterlegbar sind und wobei die wenigstens eine Übertragungseinrichtung (5) dazu geeignet und ausgebildet ist, wenigstens einen Teil der hinterlegten Therapiedaten an ein Netzwerk (16) zu übertragen **dadurch gekennzeichnet, dass** die wenigstens eine Übertragungseinrichtung (5) dazu geeignet und ausgebildet ist, wenigstens ein verfügbares Funknetz (6) zu ermitteln und wenigstens eine Kenngröße (7) für eine Netzqualität des Funknetzes (6) zu bestimmen und dass die wenigstens eine Datenverarbeitungseinrichtung (2) dazu geeignet und ausgebildet ist, eine Aufbereitung der Therapiedaten vor ihrer Übertragung in das Funknetz (6) in Abhängigkeit der Kenngröße dynamisch vorzunehmen, wobei die Therapiedaten zur Aufbereitung priorisiert werden und wobei vorrangig und/oder ausschließlich die Therapiedaten übertragen werden, welche wenigstens einen Schwellenwert für eine Priorität überschreiten.

## Claims

1. A method for operating a data processing apparatus (2) of a ventilation device (1), wherein therapy data are registered and saved in at least one storage apparatus (4) of the data processing apparatus (2) and wherein at least a part of the saved therapy data is transmitted with at least one transmission apparatus (5) to at least one network (16),
**characterized in that**
at least one available mobile network (6) is identified and at least one characteristic variable (7) for a network quality of the mobile network (6) is determined with the transmission apparatus (5) and **in that**, depending on the characteristic variable (7), at least one preparation of the therapy data is dynamically performed by the data processing apparatus (2) before the data is transmitted to the mobile network (6), wherein the therapy data are prioritized for the preparation and wherein preferentially and/or exclusively the therapy data that exceed at least one threshold for a priority are transmitted.

2. The method according to the preceding claim, wherein the characteristic variable defines an availability and/or a transmission reliability of the mobile network (6).

3. The method according to the preceding claim, wherein the threshold for the priority is dynamically established depending on the characteristic variable (7).

4. The method according to one of the two preceding claims, wherein a number of therapy hours and/or a course of the therapy (202) and/or a therapy statistic (203) are assigned a priority above the threshold.

5. The method according to one of the preceding claims, wherein the therapy data are transmitted with a redundancy that is dynamically set depending on the characteristic variable (7).

6. The method according to the preceding claim and according to one of claims 3 to 5, wherein therapy data that exceed the threshold for the priority are transmitted with a higher redundancy than therapy data below the threshold.

7. The method according to one of the preceding claims, wherein it is established depending on the characteristic variable (7) that the therapy data are transmitted over at least one network connection (26) other than the mobile network (6).

8. The method according to one of the preceding claims, wherein at least one remote setting ability of the ventilation device (1) is restricted and/or expanded depending on the characteristic variable (7).

9. The method according to one of the preceding claims, wherein, to prepare them, the therapy data are checked for changes and wherein preferentially and/or exclusively changed therapy data are transmitted.

10. The method according to one of the preceding claims, wherein at least one device parameter (201) and in particular the number of therapy hours is cumulatively counted and transmitted.

11. The method according to one of the preceding claims, wherein the preparation of the therapy data of a period in which an expected confirmation of receipt regarding the received therapy data by the mobile network (6) fails to appear comprises forming an average and wherein only the average is transmitted for at least a part of the therapy data and in particular for a course of the therapy (202) and/or a therapy statistic (203).

12. The method according to one of the preceding claims, wherein the connection of the transmission apparatus (5) to the network (6) is established at a dynamic pace and wherein the pace is specifically increased for remote setting of the ventilation device (1) and/or for an initial phase of therapy.

13. The method according to one of the preceding claims, wherein the establishment of the connection of the transmission apparatus (5) to the mobile network (6) is initiated by at least one downlink to the ventilation device (1) from a network connection (26) other than the mobile network (6) and/or is initiated by at least one user input.

14. The method according to one of the preceding claims, wherein the operation and in particular a frequency selection of the transmission apparatus (5) takes place considering at least one regional specification of the mobile network (6) and wherein the specification is identified using at least one method step from a group of method steps comprising: at least one carrier sense (listen before talk), receiving and evaluating at least one GPS signal, evaluating at least one GPS identifier of at least one base station, taking into account at least one user input, taking into account at least one regionally specific device configuration.

15. A ventilation device (1) with at least one ventilation apparatus (100) for generating an air flow for respiratory therapy, at least one data processing apparatus (2), at least one storage apparatus (4), and at least one transmission apparatus (5), wherein therapy data can be registered and can be saved in the at least one storage apparatus (4) using the at least one data processing apparatus (2) and wherein the at least one transmission apparatus (5) is suitable and designed to transmit at least a part of the saved therapy data to a network (16), **characterized in that** the at least one transmission apparatus (5) is suitable and designed to identify at least one available mobile network (6) and to determine at least one characteristic variable (7) for a network quality of the mobile network (6) and **in that** the at least one data processing apparatus (2) is suitable and designed to dynamically perform, depending on the characteristic variable, a preparation of the therapy data before their transmission to the mobile network (6), wherein the therapy data are prioritized for the preparation and wherein preferentially and/or exclusively the therapy data that exceed at least one threshold for a priority are transmitted.

## Revendications

1. Procédé de fonctionnement d'un dispositif de traitement de données (2) d'un respirateur (1), dans lequel des données thérapeutiques sont enregistrées et déposées dans un au moins un dispositif de stockage (4) du dispositif de traitement de données (2) et dans lequel une partie au moins des données thérapeutiques déposées est transmise à au moins un réseau (16) à l'aide d'au moins un dispositif de transmission (5),
**caractérisé en ce que**
le dispositif de transmission (5) permet de détecter au moins un réseau radio (6) disponible et de déterminer au moins une grandeur caractéristique (7) pour une qualité de réseau du réseau radio (6) et **en ce que** le dispositif de traitement de données (2) permet d'effectuer au moins une préparation des données thérapeutiques dynamiquement en fonction de la grandeur caractéristique (7) avant leur transmission dans le réseau radio (6), dans lequel les données thérapeutiques sont priorisées pour la préparation et dans lequel les données thérapeutiques dépassant au moins une valeur seuil pour une priorité sont transmises en premier et/ou en exclusivité.

2. Procédé selon la revendication précédente, dans lequel la grandeur caractéristique définit une disponibilité et/ou une fiabilité de transmission du réseau radio (6).

3. Procédé selon la revendication précédente, dans lequel la valeur seuil pour la priorité est fixée dynamiquement en fonction de la grandeur caractéristique (7).

4. Procédé selon l'une des deux revendications précédentes, dans lequel une priorité dépassant la valeur seuil est attribuée à un nombre d'heures de thérapie et/ou à un déroulement de thérapie (202) et/ou à une statistique de thérapie (203).

5. Procédé selon l'une des revendications précédentes, dans lequel les données thérapeutiques sont transmises avec une redondance réglée dynamiquement en fonction de la grandeur caractéristique (7).

6. Procédé selon la revendication précédente et selon l'une des revendications 3 à 5, dans lequel des données thérapeutiques dépassant la valeur seuil pour la priorité sont transmises avec une redondance plus élevée que des données thérapeutiques en dessous de la valeur seuil.

7. Procédé selon l'une des revendications précédentes, dans lequel, en fonction de la grandeur caractéristique (7), il est fixé que les données thérapeutiques sont transmises par au moins une autre connexion réseau (26) que le réseau radio (6).

8. Procédé selon l'une des revendications précédentes, dans lequel, en fonction de la grandeur caractéristique (7), au moins une capacité de réglage à distance du respirateur (1) est restreinte et/ou élargie.

9. Procédé selon l'une des revendications précédentes, dans lequel, pour la préparation, les données thérapeutiques sont examinées en vue de modifications et dans lequel des données thérapeutiques modifiées sont transmises en premier et/ou en exclusivité.

10. Procédé selon l'une des revendications précédentes, dans lequel au moins un paramètre d'appareil (201) et en particulier le nombre d'heures de thérapie est compté de façon cumulative et transmis.

11. Procédé selon l'une des revendications précédentes, dans lequel la préparation des données thérapeutiques d'une période durant laquelle une confirmation de réception attendue par le réseau radio (6) concernant les données thérapeutiques reçues fait défaut comporte la formation d'une valeur moyenne et dans lequel seule la valeur moyenne est transmise pour une partie au moins des données thérapeutiques et en particulier pour un déroulement de thérapie (202) et/ou une statistique de thérapie (203).

12. Procédé selon l'une des revendications précédentes, dans lequel l'établissement de la connexion du dispositif de transmission (5) au réseau (6) est effectué selon une synchronisation dynamique et dans lequel la synchronisation est augmentée de façon ciblée pour un réglage à distance du respirateur (1) et/ou une phase de thérapie initiale.

13. Procédé selon l'une des revendications précédentes, dans lequel l'établissement de la connexion du dispositif de transmission (5) au réseau radio (6) est initié par au moins une liaison descendante au respirateur (1) à partir d'une autre connexion réseau (26) que le réseau radio (6) et/ou est initié par au moins une entrée d'utilisateur.

14. Procédé selon l'une des revendications précédentes, dans lequel l'exploitation et en particulier une sélection de fréquence du dispositif de transmission (5) tient compte d'au moins un paramètre régional du réseau radio (6) et dans lequel le paramètre est déterminé à l'aide d'au moins une étape de procédé parmi un groupe d'étapes de procédé, comportant : au moins une détection de porteuse (écouter avant de parler), la réception et l'évaluation d'au moins un signal GPS, l'évaluation d'au moins un identifiant GPS d'au moins une station de base, la prise en compte d'au moins une entrée d'utilisateur, la prise en compte d'au moins une configuration d'appareil régionale spécifique.

15. Respirateur (1) comprenant au moins un dispositif respiratoire (100) destiné à produire un flux d'air pour une thérapie respiratoire, au moins un dispositif de traitement de données (2), au moins un dispositif de stockage (4), et au moins un dispositif de transmission (5), dans lequel des données thérapeutiques peuvent être enregistrées à l'aide de l'au moins un dispositif de traitement de données (2) et déposées dans l'au moins un dispositif de stockage (4) et dans lequel l'au moins un dispositif de transmission (5) est adapté et conçu pour transmettre au moins une partie des données thérapeutiques enregistrées à un réseau (16), **caractérisé en ce que** l'au moins un dispositif de transmission (5) est adapté et conçu pour détecter au moins un réseau radio (6) disponible et pour déterminer au moins une grandeur caractéristique (7) pour une qualité de réseau du réseau radio (6) et **en ce que** l'au moins un dispositif de traitement de données (2) est adapté et conçu pour effectuer une préparation des données thérapeutiques dynamiquement en fonction de la grandeur caractéristique avant leur transmission dans le réseau radio (6), dans lequel les données thérapeutiques sont priorisées pour la préparation et dans lequel les données thérapeutiques dépassant au moins une valeur seuil pour une priorité sont transmises en premier et/ou en exclusivité.
